# EUROPEAN PATENT APPLICATION

(11) **EP 3 087 853 A1**
(43) Date of publication of application: **02.11.2016**
(21) Application number: 15165352.4
(22) Date of filing: 28.04.2015
(51) Int. Cl.: A24F 47/00

(54) **ELECTRONIC SMOKING DEVICE AND CARTOMIZER**

(71) Applicant: Fontem Holdings 1 B.V., 1083 HN Amsterdam (NL)
(72) Inventor: Borkovec, Vaclav, 22761 Hamburg (DE)
(74) Representative: Gulde & Partner

(57) **Abstract**

An cartomizer (40) adapted for an electronic smoking device (10), comprises a shell(41) having an air inhalation port (36) in a first end and a coupling (42) adapted to couple to a main body (12) of the electronic smoking device (10) at an opposite second end; an atomizer (26) operable to create an aerosol for inhalation by a user through the air inhalation port (36) by atomizing a liquid supplied from a liquid store (34) arranged inside the shell (41); and a first sealing member (44) sealing the mouthpiece opening (36) and a second sealing member (46) sealing the second end.

## Description

### FIELD OF INVENTION

The present invention relates generally to electronic smoking devices and in particular a cartomizer and electronic cigarettes.

### BACKGROUND OF THE INVENTION

An electronic smoking device, such as an electronic cigarette (e-cigarette), usually has a housing accommodating an electric power source (e.g. a single use battery or a rechargeable battery), and an electrically operable atomizer. The atomizer vaporizes or atomizes liquid supplied from a reservoir and provides vaporized or atomized liquid as an aerosol. Control electronics control the activation of the atomizer. In many electronic cigarettes, an airflow sensor is provided within the electronic smoking device which detects a user puffing on the device (e.g., by sensing an under-pressure or an air flow pattern through the device). The airflow sensor indicates or signals the puff to the control electronics. Alternatively, a button may be used to switch on the electronic smoking device to generate a puff of flavour. When a puff is detected, the control electronics supplies electrical power to the atomizer thereby creating vaporized liquid as an aerosol.

A cartomizer is available for many e-cigarettes. The cartomizer replaces the otherwise separate cartridge and atomizer components with a single integrated component, hence the nomenclature cartomizer. The cartomizer is usually disposable, as opposed to stand-alone atomizers that are reusable and comparatively expensive.

### SUMMARY OF THE INVENTION

In accordance with one aspect of the present invention there is provided a cartomizer adapted for an electronic smoking device. The cartomizer comprises a shell having an air inhalation port and a coupling adapted to couple to a main body of the electronic smoking device at an opposite second end. The cartomizer further comprises an atomizer operable to create an aerosol for inhalation by a user through the air inhalation port by atomizing or vaporizing a liquid supplied from a liquid store arranged inside the shell. The cartomizer comprise a first sealing member sealing the mouthpiece opening and a second sealing member sealing the second end.

The characteristics, features and advantages of this invention and the manner in which they are obtained as described above, will become more apparent and be more clearly understood in connection with the following description of exemplary embodiments, which are explained with reference to the accompanying drawings.

### BRIEF DESCRIPTION OF THE DRAWINGS

In the drawings, same element numbers indicate same elements in each of the views:
Figure 1 is a schematic cross-sectional illustration of an exemplary e-cigarette;
Figure 2 is a schematic cross-sectional illustration of an embodiment of a cartomizer of the exemplary e-cigarette.
Figure 3 is a schematic cross-sectional illustration of a further embodiment of a cartomizer of the exemplary e-cigarette.
Figure 4 is a schematic cross-sectional illustration of an even further embodiment of a cartomizer of the exemplary e-cigarette.
Figure 5 is a schematic cross-sectional illustration of the exemplary e-cigarette before connection of the cartomizer and a main body.

### DESCRIPTION OF THE PREFERRED EMBODIMENTS

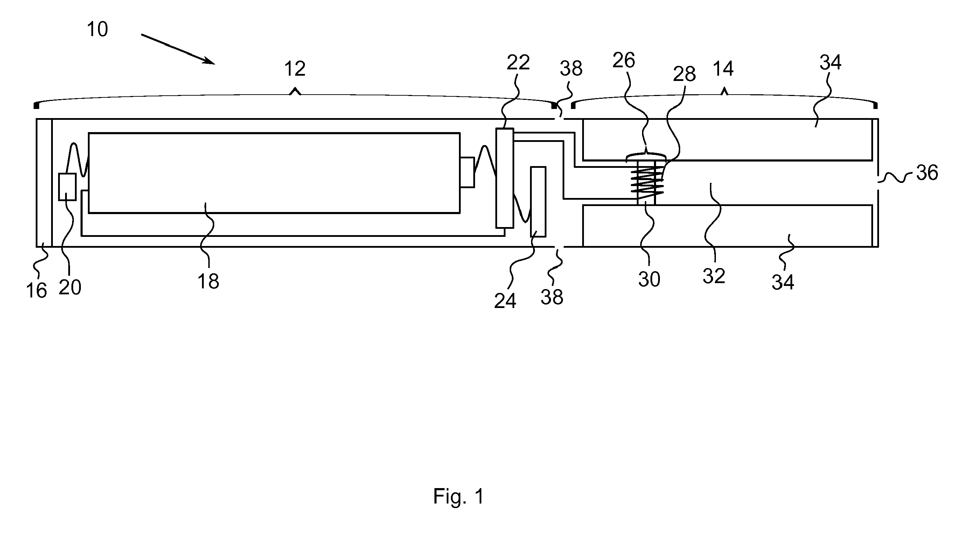

Prior to describing an embodiment of the present invention, the basic structure of an e-cigarette will first be described with reference to Figure 1 which is a schematic cross-sectional illustration of an exemplary e-cigarette.

As is shown in Figure 1, an e-cigarette 10 ordinarily comprises a cylindrical housing having a main body 12 and a mouthpiece portion 14. Together the main body 12 and the mouthpiece portion 14 form a cylindrical tube which is approximately the same size and shape as a conventional cigarette. The main body 12 and mouthpiece 14 are typically made of steel or hardwearing plastic and act to provide a housing to contain the operative elements of the e-cigarette 10. The main body 12 and a mouthpiece portion 14 may be configured to fit together by means of a friction push fit. Alternatively in some embodiments a screw fit may be provided enabling the main body 12 and mouthpiece portion 14 to be attached to one another. Alternatively in some e-cigarettes the main body 12 and mouthpiece portion 14 may be parts of a single integrally formed tube.

An end cap 16 is provided at the end of the main body 12 remote from the mouthpiece portion 14 enclosing that end of the main body 12. The end cap 16 is typically made from translucent plastic.

A battery 18 is provided within the central cavity enclosed by the main body 12. Also contained within the central cavity defined by the main body 12 are a light emitting diode (LED) 20, control electronics 22 and an airflow sensor 24. The battery 18 is electrically connected to the LED 20 and the control electronics 22 and the airflow sensor 24 is connected to the control electronics 22. In this example the LED 20 is provided at one end of the main body 12, adjacent to the end cap 16 and the control electronics 22 and airflow sensor 24 are provided in the central cavity at the other end of the battery 18 adjacent the mouth piece portion 14.

The airflow sensor 24 acts as a puff detector, detecting a user puffing or sucking on the mouthpiece portion 14 of the e-cigarette 10. The airflow sensor 24 can be any suitable sensor for detecting changes in airflow or air pressure such a microphone switch including a deformable membrane which is caused to move by variations in air pressure.

The control electronics 22 are also connected to an atomizer 26 which in this illustrative example comprises a heating coil 28 which is wrapped around a wick 30 which extends across a central passage 32 provided in the mouthpiece portion 14 of the e-cigarette 10. The dimensions of the central passage 32, the wick 30 and the heating coil 28 are such that the wick 30 and heating coil 28 do not completely block the central passage 32 but rather an air gap is provided at either side of the heating coil 28 enabling air to flow past the heating coil 28 and the wick 30.

The central passage 32 is surrounded by a cylindrical liquid store 34 with the ends of the wick 30 abutting or extending into the liquid store 34. The wick 30 comprises a porous material such as a bundle of fibreglass fibres such that liquid present in the liquid store 34 is drawn by capillary action from the ends of the wick 30 towards the central portion of the wick 30 encircled by the heating coil 28.

In some embodiments the liquid store 34 will comprise wadding soaked in liquid which encircles the central passage 32 with the ends of the wick 30 abutting the wadding. In other embodiments the liquid store 34 may comprise a toroidal cavity arranged to be filled with liquid to be vaporized with the toroidal cavity enclosed by walls and with the ends of the wick 30 extending into the toroidal cavity.

An air inhalation port 36 is provided at the end of the mouthpiece portion 14 remote from main body 12 of the e-cigarette 10 and a pair of air inlets 38 are provided in the housing at the intersection between the main body 12 and the mouthpiece portion 14 adjacent the airflow sensor 24 with the central passage 32 within the mouthpiece portion 14 of the e-cigarette 10 extending from adjacent the air inlets 38 past the wick 30 and heating coil 28 to the air inhalation port 36.

In use, a user sucks on the mouthpiece portion 14 of the e-cigarette 10. This causes air to be drawn into the e-cigarette 10 via the air inlets 38 and to be drawn up via the central passage 32 towards the air inhalation port 36. The change in air pressure which arises is detected by the airflow sensor 24 which generates an electrical signal that is passed to the control electronics 22. In response to the signal, the control electronics 22 then proceed to activate the heating coil 28 which causes liquid present in the wick 30 to be vaporized creating an aerosol (which may comprise gaseous and liquid components) within the central passage 32. As the user continues to suck on the mouthpiece portion 14 of the e-cigarette 10, this aerosol is drawn up the central passage 32 and inhaled by the user sucking on the e-cigarette 10. At the same time the control electronics 22 also activate the LED 20 causing the LED 20 to light up which is visible via the translucent end cap 16 mimicking the appearance of a glowing ember at the end of a conventional cigarette. As liquid present in the wick 30 is converted into an aerosol more liquid is drawn into the wick 30 from the liquid store 34 by capillary action and thus is available to be converted into an aerosol through subsequent activation of the heating coil 28.

In some e-cigarettes, the e-cigarette 10 is intended to be disposable and the electric power in the battery 18 is intended to be sufficient to vaporize the liquid contained within the liquid store 34 after which the e-cigarette 10 is thrown away. In other embodiments the battery 18 is rechargeable and a means is provided to replenish the liquid supply. In the cases where the liquid store 34 is a toroidal cavity, this may be achieved by providing a refill port and refilling the cavity with liquid via the refill port. In other embodiments the mouthpiece portion 14 of the e-cigarette 10 is detachable from the main body 12 and a new mouthpiece portion 14 can be fitted with a new liquid store 34 thereby replenishing the supply of liquid. In some cases, replacing the liquid store 34 may involve replacement of the heating coil 28 and the wick 30 along with the replacement of the liquid store 34.

In some cases the new liquid store 34 may be in the form of a cartridge. In some such embodiments the cartridge may be provided with a central passage 32 through which a user inhales aerosol generated by the e-cigarette. In other embodiments, rather than inhaling aerosol via a central passage 32, the cartridge may be such to block the central portion of the e-cigarette 10 and generated aerosol may be directed around the exterior of the cartridge 32 to an air inhalation port 36 for inhalation.

It will also be appreciated that although the above description is illustrative of the structure and function of a typical e-cigarette 10, variations also exist. Thus for example in some e-cigarettes the LED 20 is omitted. In some e-cigarettes, the airflow sensor 24 may be placed adjacent the end cap 16 of the e-cigarette rather than in the middle of the e-cigarette as illustrated. Similarly, in some e-cigarettes, the air inlets 36 may be placed at the distal end of the main body 16 of the e-cigarette 10 remote from the mouthpiece portion 14. In some e-cigarettes the airflow sensor 24 is omitted and instead a button is provided which enables a user to activate the e-cigarette manually rather than in response to the detection of a change in air flow or air pressure. Also in some e-cigarettes the constitution of the atomizer may be changed. Thus for example rather than being constituted by a wick 28 surrounded by a heating coil 26 other configurations may be used such as providing a heating coil in the interior of a porous body and generating an aerosol by heating air and passing the air through the porous body. Further in some embodiments rather than generating an aerosol through heating liquid within a wick 28 an aerosol might be generated using a piezoelectric atomizer to create an aerosol for inhalation either in combination or in the absence of a heater.

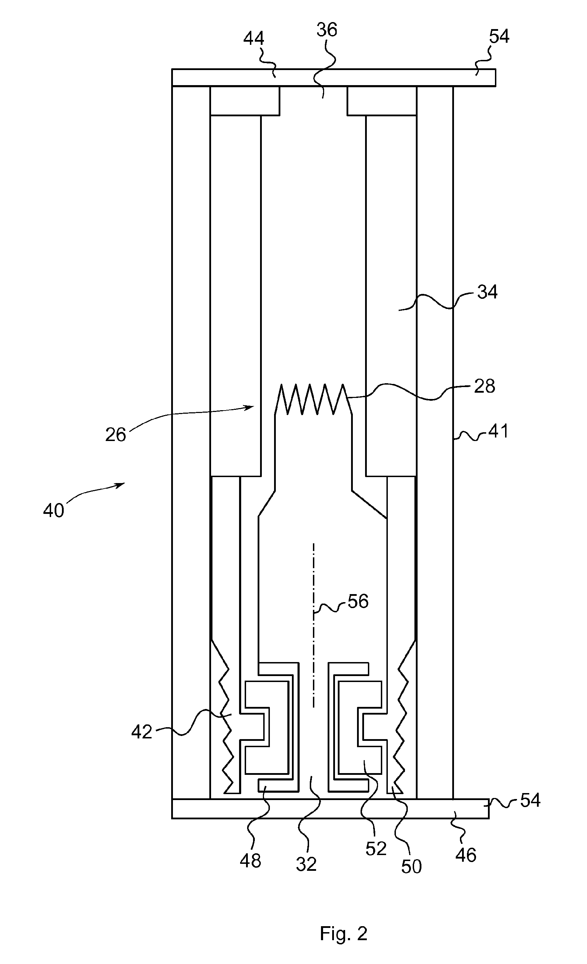

In Figure 2 a first embodiment of a cartomizer 40 is shown. The cartomizer 40 corresponds to the mouthpiece portion 14 of the e-cigarette 10 shown in Figure 1. The cartomizer 40 has a tubular outer shell 41 having an air inhalation port 36 in a first end and a coupling 42 adapted to couple to the main body 12 of the electronic smoking device 10. The coupling 42 is arranged at a second end which is opposite to the first end. The cartomizer 40 further includes an atomizer 26 operable to create an aerosol for inhalation by the user through the mouthpiece opening 36 by atomizing or vaporizing a liquid supplied from a liquid store 34 arranged inside the shell 41 where the atomizer 26 is contained within a central passage 32 provided within a cylindrical liquid store 34. For ease of understanding the atomizer 26 with its coil 28 and the liquid store 34 are depicted only in a schematic way.

In contrast to a conventional cartomizer, a first sealing member 44 is provided sealing the air inhalation port 36, and a second sealing member 46 seals the other end of the cartomizer.

In a conventional cartomizer, an atomizer 26 is in direct contact with air within the central passage 32 which is in direct communication with the atmosphere via the air inhalation port 36 and an opening at the other end of the cartomizer. This means that liquid which is wicked from the liquid store 34 adjacent the atomizer 26 is liable to evaporate or become contaminated with water vapour absorbed from the atmosphere.

The barrier membranes or sealing members 44 and 46 prevent water absorption and nicotine migration thereby protecting the liquid. The sealed cartomizer 40 and with it the sealed liquid storage 34 lengthens the shelf life of the cartomizer 40. As the liquid is protected the total volume of liquid inside the liquid storage 34 is kept constant thereby keeping the ratio between flavoured materials and liquid constant. Such provision is keeping the dosage of the cartomizer 40 at least more constant. This allows registering the device as medical product.

The cartomizer 40 comprises a first electrical contact 48 and a second electrical contact 50 to allow for electrical connection of the cartomizer 40 to the main body 12. To be more specific, the electrical contacts 48 and 50 allow for powering the atomizer 26 by the battery 18 of the main body 12. The first contact 48 has a toroidal body arranged in the center of the cartomizer 40 at its second end. In its central portion the first contact 48 defines the central passage 32. Through the central passage 32 air flows through the atomizer 26 and finally to the air inhalation port 36. An insulator 52 having a toroidal body is arranged between the first contact 48 and the second contact 50 in order to electrically insulate the two electrical contacts 48 and 50. The second contact 50 is part of the coupling 42 which is realized in this embodiment as a male or external thread. The coupling 42 is fixed inside the cartomizer 40 for example by gluing, pressing or other suitable methods. The coupling 42 may consist of plastic or metal. The second contact 50 may be realized distinct from the coupling 42 so that the coupling 42 needs not to be electrically conductive.

Instead of a threaded coupling 42 other types of coupling like bayonet coupling, snap coupling or the like may be implemented.

One or more of the sealing members 44 and 46 may consist of a metal foil like for example aluminum foil. Such a seal or barrier offers a low permeability and easy attachment to the cartomizer 40. Alternatively, a plastic foil or sheet of PET or PE for example may be used. Any material suitable to prevent nicotine and water or moisture migration may be used for the sealing member 44 or 46.

Such a metal foil may further be coated or laminated with a plastic material. Such a material may ease connection between the cartomizer 40 and the sealing member 44 or 46. Preferably, the plastic material of the sealing member 44 or 46 is identical or similar to that of the shell 41. Such a configuration improves connection between those elements.

One or both of the sealing members 44 and 46 may comprise a grip or tag for easy removal of the sealing member by the user prior to use of the cartomizer 40. Such a grip 54 eases removal of the sealing member.

When the first sealing member 44 is attached to the first end of the cartomizer shell 41, the grip 54 projects over the periphery of the shell 41 so that it can be grasped by a user. Alternatively, the first sealing member 44 may be aligned with the outer diameter of the shell 41. In an alternative the first sealing member 44 may have a diameter which is smaller than the diameter of the shell 41 but larger than the diameter of the air inhalation port 36. In a further alternative, the first sealing member 44 may have a diameter larger than the diameter of the shell 41. In such case the first sealing member 44 may be flanged to the outside of the mouthpiece shell 41. The second sealing member 46 is attached to the opposite end of the cartomizer 40. The coupling 52, the central passage 32 and the two contacts 48 and 50 are covered by the second sealing member 46. Preferably none of these parts is in contact with the second sealing member 46. In other words, the coupling 42 and the electrical contacts 48 and 50 are positioned distanced away from the second sealing member 46. In such an arrangement the cartomizer shell 41 extends past the coupling 42 at the second end in a direction of a central axis 56. This has the advantage that both sealing members 44 and 46 are attached to the shell 41 so that the same process for attaching the two sealing members 44 and 46 may be used.

The first sealing member 44 and/or the second sealing member 46 may be heat-sealed to the shell 41. Such a heat-sealing process offers a durable and leak-proof connection between the sealing member 44 or 46 and the shell 41. Depending on the materials of the shell 41 and the respective sealing member 44 or 46 one of various heat-sealing methods or connections may be used. For example, the heat-sealing member is heat-sealed according to one of a metal on plastic, a metal on glass, a plastic on plastic, a plastic on a glass and a metal on metal connection. In one example the body may consist of plastic material and the sealing member 44 or 46 may consist of a metal foil or a metal foil laminated with plastic. For such example a metal on plastic or plastic on plastic heat-sealed connection is achieved. In another example the shell 41 may consist of metal and the sealing member 44 or 46 may consist of a metal foil or a metal foil laminated with plastic material. Accordingly, the heat-sealing connection is of the type metal on plastic or metal on metal. In an even further example the body may consist of glass and the sealing member 44 or 46 may consist of a metal foil or a metal foil laminated with plastic. The heat-sealing process is accordingly a metal on glass or plastic on glass process.

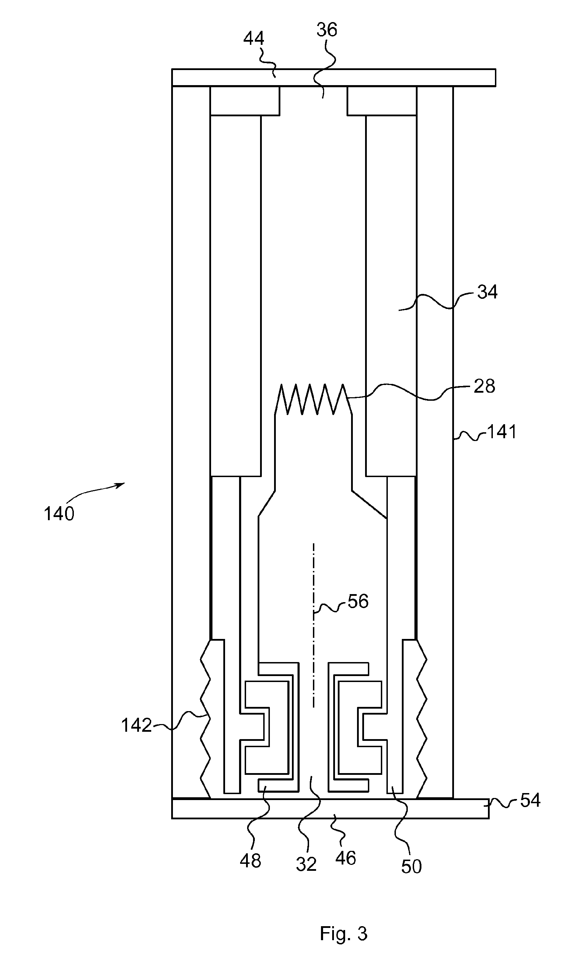

Figure 3 shows a further embodiment of the cartomizer 140. As in Figure 2, the air inhalation port 36 is sealed by a first sealing member 44 and the central passage 32 is sealed by the second sealing member 46. The two sealing members 44 and 46 effectively seal the liquid store 34. Accordingly, water absorption by the liquid and nicotine migration from the liquid is beneficially prevented.

In contrast to Figure 2 the embodiment shown in Figure 3 has a coupling 142 in form of an internal or female thread. The female thread of the coupling 142 is arranged at an inner surface of the shell 141. The thread and the coupling 142 may be formed integrally with the shell 141 as shown in Figure 3. Alternatively, a coupling with its female thread may have the form of a sleeve or tube and is arranged on the inside of the shell. Here, the second contact 50 has only the functionality of an electric contact as the coupling 142 is integrated into the shell 141. The electrical contracts 48 and 50 are spaced apart from the second sealing member 46. The coupling 142 is covered at its distal end by the second sealing member 46. Depending on the definition the second sealing member 46 is attached to the shell 141 or to the coupling 142.

In conjunction with Figure 3 only the differences with regard to Figure 2 have been described. The cartomizer 40 as shown in Figure 2 is compatible to a main body 12 having a female or internal thread as the counter part to the coupling 42. The cartomizer 140 as shown in Figure 3 is compatible to a main body 12 having an external or male thread as a counter part to the coupling 142.

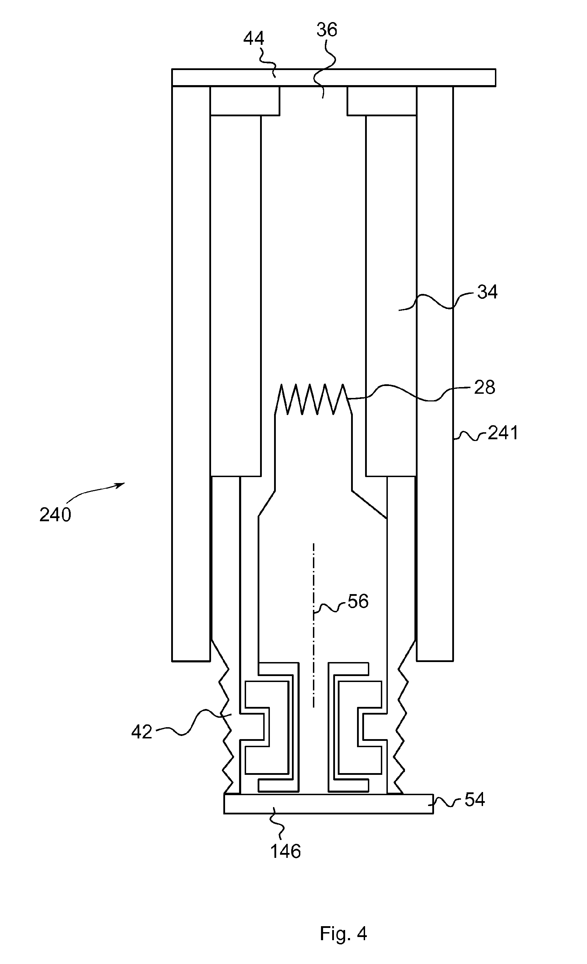

Figure 4 shows a further embodiment of cartomizer 240. The cartomizer 240 has an external or male threading 42 like the cartomizer shown in Figure 2. The first end or mouthpiece opening 36 of the cartomizer 240 is sealed again by the first sealing member 44.

Here, the coupling 42 extends past the shell 241 at the second end of the cartomizer 240 in a direction along the central axis 56. In accordance with such configuration a second sealing member 146 is attached to a front side or distal end of the coupling 42. The second sealing member 146 may also comprise a flap or grip 54. The diameter of the second sealing member 146 is adapted to the diameter of the coupling 42. Hence, the diameter of the second sealing member 146 is smaller than the diameter of the first sealing member 44. The second sealing member 146 is attached or fastened to the coupling 42 according to one of the methods as described in conjunction with Figure 2. Preferably the coupling 42 consists of plastic and more preferably the coupling 42 consists of metal. Hence, the second sealing member 146 is preferably heat-sealed according to a metal on plastic, a plastic on plastic or a metal on metal connection or process.

The use of the cartomizer 40, 140 or 240 will now be described.

In use, a user obtains an electronic smoking device 10, here in form of an e-cigarette. The e-cigarette 10 has a main body 12 with an electric power source 18, here in form of a battery, for powering the atomizer 26. The e-cigarette 10 further includes at least one cartomizer 40, 140 or 240. Prior to connection of the cartomizer 40, 140 or 240 with the main body 12 the user removes the first sealing member 44 and the second sealing member 46 or 146. Advantageously, the user grabs the grips 54 and pulls of the sealing members. The order of pulling off the sealing members is unimportant. The user could however, remove the second sealing member 46 or 146 and connect the cartomizer with a main body 12 by the coupling 42 or 142 and leave the first sealing member 44 still attached to the first end of the cartomizer. This way, the user has an assembled e-cigarette 10 having its liquid at least partly protected by the first sealing member 44 being still in place. Prior to the actual use of the e-cigarette 10 the user than peels off the first sealing member 44.

When the liquid stored in liquid store 34 is depleted the user disconnects the coupling 42 thereby removing the cartomizer 40, 140 or 240 from the main body 12. In order to prepare the e-cigarette 10 for use again the user obtains a sealed cartomizer 40,140, 240 from its stock or a shop. The two sealing members protect the liquid inside the liquid storage 34 during storage of the cartomizer. This ensures that the liquid of the cartomizer keeps its quality and quantity of the time of production during a long storage time.

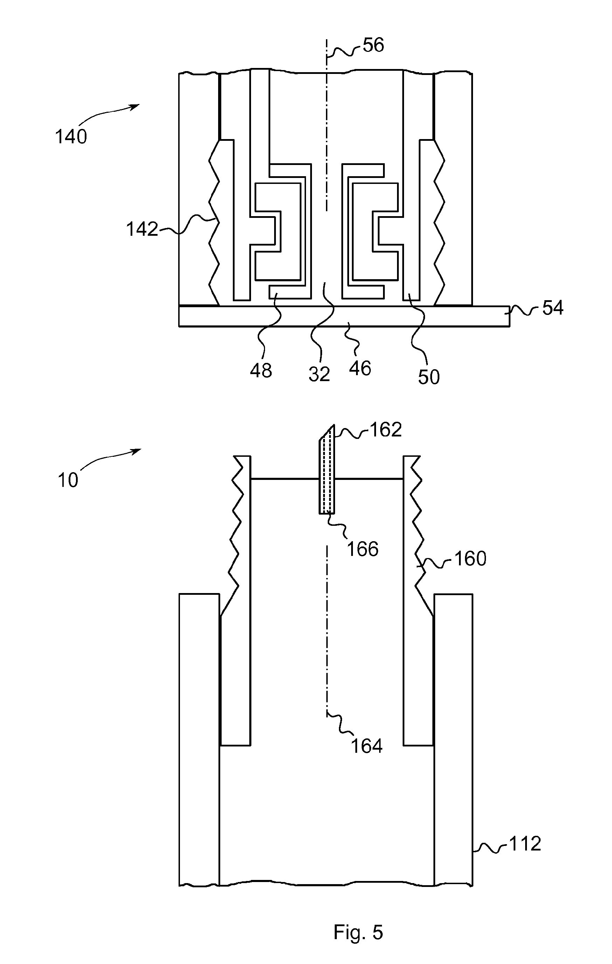

Figure 5 shows an alternative way of coupling the atomizer with a main body 12. While in Figure 5 the cartomizer 140 of Figure 3 is depicted the same connection is possible with the cartomizer 40 of Figure 2 or the cartomizer 240 of Figure 4.

According to this embodiment the second sealing member 46 is breached or pierced automatically while the cartomizer 140 and the main body 112 are connected. Such a connection has the advantage of easier handling as the second sealing member 46 does not need to be removed prior to assembly of the e-cigarette 10. The second sealing member 46 however could still comprise a grip 54 so that user may remove the second sealing member 46 prior to use if desired.

The main body 112 comprises a coupling 160 which is the counterpart to the coupling 142 of the cartomizer 140. In this example the coupling 160 is configured as an external or male thread. In order to assemble the e-cigarette 10, i.e. to connect the cartomizer 140 and the main body 112 the two parts are aligned along their axes 56 and 164 and screwed together.

The main body 112 has a piercing member 162 which is arranged in a middle or central portion of the main body 112. The piercing member 162 may consist of plastic or metal. Preferably, the piercing member 162 is arranged at the central axis 164 of the main body 112. During connection of the cartomizer 140 and the main body 112 the central axes 56 and 164 are aligned. This implies that the piercing member 162 is orientated towards the central passage 32 of the cartomizer 140.

The piercing member 162 or a tip of the piercing member 162 may extend past the coupling 160 in an axial direction. It may also be possible that the piercing member 162 is aligned with the coupling 160 or that the piercing member 162 rejects past the coupling 160. Either way, the piercing member is adapted to pierce the second sealing member 46 upon connection of the main body 112 and the cartomizer 140. Depending on the axial location of the piercing member 162 the piercing of the second sealing member 46 may take place at the beginning, in the middle or at the end of the connection process.

Prior, during or after connection of the cartomizer 140 and the main body 112, the second sealing member 46 is pierced thereby allowing airflow from a main body 112 through the central passage 32.

Preferably, the location and/or the length in axial direction of the piercing member 162 is adapted so that it is bridging the second sealing member 46 in the assembled state of the e-cigarette 10. Further, the piercing member 162 has a hollow interior or passage 166. Such configuration guarantees airflow through the passage 166 of the piercing member 162 even when the second sealing member 46 has the intention to close again after it has been pierced by the piercing member 162.

In Figure 5 the main body 112 is depicted in a schematic way for illustration of the piercing member 162 and the process of connecting the cartomizer 140 with the main body 112. Further parts of the main body 112 like control electronics 22, the airflow sensor 24 or electrical contacts have been omitted for the sake of clarity. The attachment or installation of the piercing member 162 is also depicted in a schematic way. The piercing member 162 may be attached to or be part of the coupling 160, a housing of the main body 112 or electrical contacts (not shown) of the main body 112.

## Claims

1. A cartomizer adapted for an electronic smoking device (10), comprising:
- a shell (41) having an air inhalation port (36) in a first end and a coupling (42) adapted to couple to a main body (12) of the electronic smoking device (10) at an opposite second end;
- an atomizer (26) operable to create an aerosol for inhalation by a user through the air inhalation port (36) by atomizing a liquid supplied from a liquid store (34) arranged inside the shell (41);
- a first sealing member (44) sealing the mouthpiece opening (36); and
- a second sealing member (46) sealing the second end.

2. The cartomizer according to claim 1, wherein at least one sealing member (44, 46) comprises of a metal foil.

3. The cartomizer according to claim 2, wherein the metal foil is coated with plastic material.

4. The cartomizer according to one of claims 1 to 3, wherein at least one sealing member (44, 46) comprises an aluminium foil.

5. The cartomizer according to one of claims 1 to 4, wherein at least one sealing member (44, 46) is heat-sealed to the shell (41).

6. The cartomizer according to claim 5, wherein at least one sealing member (44, 46) is heat-sealed according to one of a metal on plastic, a metal on glass, a plastic on plastic, a plastic on glass and a metal on metal connection.

7. The cartomizer according to one of claims 1 to 6, wherein the coupling (42) extends past the shell (41) at the second end and wherein the second sealing member (46) is attached to the coupling (42).

8. The cartomizer according to one of claims 1 to 6, wherein the shell (41) extends past the coupling (42) at the second end and wherein the second sealing member (46) is attached to the body (14).

9. The cartomizer according to one of claims 1 to 8, wherein at least one sealing member (44, 46) comprises a grip (54) adapted for removal of the sealing member (44, 46).

10. The cartomizer according to one of claims 1 to 9, wherein the shell (41) consists of at least one of plastic, metal and glass.

11. The cartomizer according to one of claims 1 to 10, wherein the coupling (42) comprises plastic or metal.

12. The electronic cigarette according to one of claims 1 to 11, wherein the coupling (42) comprises a male threading or a female threading arranged at an inner surface of the shell (41).

13. An electronic smoking device comprising a main body (12) with an electric power source (18) for powering an atomizer (26) and a cartomizer (40) according to one of claims 1 to 12.

14. The electronic smoking device according to claim 13, wherein a piercing member (162) is adapted to pierce the second sealing member (46) upon connection of the main body (12) and the cartomizer (40).

15. The electronic smoking device according to claim 14, wherein the piercing member (162) comprises a hollow needle.
